# EUROPEAN PATENT APPLICATION

(11) **EP 3 305 312 A1**
(43) Date of publication of application: **11.04.2018**
(21) Application number: 16803376.9
(22) Date of filing: 01.06.2016
(51) Int. Cl.: A61K 38/00, A61K 9/08, A61K 47/10, A61K 47/14, A61P 17/00, A61P 17/06, A61P 17/14, A61P 37/06, A61P 37/08

(54) **EXTERNAL PREPARATION FOR TRANSDERMAL ADMINISTRATION**

(30) Priority: 05.06.2015 JP 2015114417
(71) Applicant: Maruho Co., Ltd., Osaka 531-0071 (JP)
(72) Inventor: SAKIYAMA, Hiroki, osaka-shi Osaka 531-0071 (JP); KANEDA, Noriaki, osaka-shi Osaka 531-0071 (JP)
(74) Representative: Hoffmann Eitle
(86) International application number: PCT/JP2016/066174
(87) International publication number: WO 2016/194942

(57) **Abstract**

The present invention is intended to provide a cyclosporine external preparation that improves transdermal absorption of cyclosporine. The present invention provides an external preparation containing cyclosporine, ethanol, and a fatty acid monoester.

## Description

### TECHNICAL FIELD

The present invention relates to a cyclosporine external preparation that improves transdermal absorption of cyclosporine.

### BACKGROUND ART

Cyclosporine is a compound having immunosuppressing effect and hair growth promoting effect, and is known to be effective against skin diseases such as psoriasis, atopic dermatitis, and alopecia areata. However, with a large molecular weight of about 1,200, transdermal absorption is difficult to achieve, particularly in human skin, which has a considerably thicker stratum corneum than animal skin.

There are studies directed to improving transdermal absorption of cyclosporine. Non patent literature 1 investigates a plurality of vehicles for local delivery of cyclosporine, and finds that the highest efficiency occurs with 40% ethanol.

### CITED REFERENCES

### NON PATENT LITERATURE

NON PATENT LITERATURE 1: International Journal of pharmaceutics 311 (2006) 182-186

### DISCLOSURE OF THE INVENTION

### PROBLEMS TO BE SOLVED BY THE INVENTION

Alopecia areata is a condition that involves round or patchy bald spots on the scalp or other areas of body where there is hair. Histopathologically, alopecia areata is characterized by lymphocyte infiltration around the hair follicle, and is known to be an autoimmune disease, or an autoimmune disease based on abnormal local immunity.

The previous studies of the conditions and the causes of this disease have led to the use of cyclosporine A (CyA) for the treatment of alopecia areata (unapproved). For treatment, a commercially available oral form or injection is used. However, a drawback of CyA and other members of calcineurin inhibitors is their side effects. Systemic administration of these drugs are known to cause a range of side effects, including high-blood pressure, and kidney failure. There is also a risk of drug interactions with other drugs, which may limit the use of these drugs.

By using CyA as an external preparation for local treatment, it would be possible to reduce the systemic side effects, and to locally show efficacy with an increased concentration of the active component at the affected area.

However, as mentioned above, transdermal absorption of cyclosporine is poor, and there is no known cyclosporine external preparation that can achieve sufficient transdermal absorption into human skin.

It is accordingly an object of the present invention to provide a cyclosporine external preparation that improves transdermal absorption of cyclosporine.

### MEANS FOR SOLVING THE PROBLEMS

The present inventors diligently worked to find a solution to the foregoing problem, and found that the transdermal absorption of cyclosporine can be improved by incorporating ethanol and a fatty acid monoester. The present invention has been completed on the basis of this finding.

Specifically, the present invention is as follows.
(1) An external preparation comprising cyclosporine, ethanol, and a fatty acid monoester (hereinafter referred to as "the external preparation of the present invention").
(2) The external preparation described in (1) above, wherein the fatty acid monoester is an ester of a monohydric alcohol of 1 to 22 carbon atoms and a monocarboxylic acid of 6 to 22 carbon atoms.
(3) The external preparation described in (1) above, wherein the fatty acid monoester is isopropyl myristate, isopropyl palmitate, or 2-hexadecyl isostearate.
(4) The external preparation described in (1) above, which is substantially free of water used as a solubilizer.
(5) The external preparation described in (1) above, which is a liquid formulation.

### EMBODIMENTS FOR CARRYING OUT THE INVENTION

An external preparation of the present invention contains cyclosporine as a medicament.

"Cyclosporine" according to the external preparation of the present invention is a concept that includes, for example, cyclosporine A, cyclosporine B, cyclosporine C, cyclosporine D, and cyclosporine H. The preferred cyclosporine in the present invention is cyclosporine A.

The appropriate cyclosporine content in the external preparation of the present invention is 1 weight% or more, preferably 1.25 weight% or more, more preferably 2 weight% or more, further preferably 2.5 weight% or more with respect to the total preparation amount. The appropriate cyclosporine content is 50 weight% or less, preferably 40 weight% or less, more preferably 30 weight% or less, further preferably 10 weight% or less with respect to the total preparation amount.

The appropriate cyclosporine content in the external preparation of the present invention is 1 to 50 weight%, preferably 1.25 to 40 weight%, more preferably 2 to 30 weight%, further preferably 2.5 to 10 weight% with respect to the total preparation amount.

A feature of the external preparation of the present invention is that ethanol and a fatty acid monoester are used as a solubilizer for dissolving cyclosporine.

With the solubilizer adequately dissolving cyclosporine, the external preparation of the present invention can exhibit excellent transdermal absorption.

Preferred as the ethanol used in the external preparation of the present invention is an anhydrous ethanol.

The anhydrous ethanol may be, for example, the anhydrous ethanol specified in the 16th Edition of the Japanese Pharmacopoeia.

The appropriate ethanol content in the external preparation of the present invention is 3 to 90 weight%, preferably 5 to 70 weight%, more preferably 10 to 60 weight%, further preferably 15 to 50 weight% with respect to the total preparation amount. Transdermal absorption tends to decrease when the ethanol content is less than 1 weight%. From the viewpoint of reducing local irritation, the ethanol content is preferably 50 weight% or less.

"Fatty acid monoester" according to the external preparation of the present invention means an ester of an alcohol and an aliphatic monocarboxylic acid.

The alcohol is not particularly limited, as long as it is pharmaceutically acceptable. Examples of suitable alcohols include monohydric alcohols of 1 to 22 carbon atoms, preferably monohydric alcohols of 1 to 16 carbon atoms, more preferably monohydric alcohols of 1 to 3 carbon atoms.

Examples of the monohydric alcohols of 1 to 22 carbon atoms include methyl alcohol, ethyl alcohol, propyl alcohol, isopropyl alcohol, lauryl alcohol, myristyl alcohol, palmityl alcohol, stearyl alcohol, isostearyl alcohol, cetostearyl alcohol, 2-hexadecyl alcohol, octyldodecyl alcohol, and behenyl alcohol.

The aliphatic monocarboxylic acid is not particularly limited, as long as it is pharmaceutically acceptable. Examples of suitable aliphatic monocarboxylic acids include monocarboxylic acids of 6 to 22 carbon atoms, preferably monocarboxylic acids of 14 to 16 carbon atoms.

Examples of the monocarboxylic acids of 6 to 22 carbon atoms include caproic acid, caprylic acid, capric acid, lauric acid, myristic acid, palmitic acid, stearic acid, behenic acid, and isostearic acid.

Examples of the fatty acid monoester include isopropyl myristate, isopropyl palmitate, and 2-hexadecyl isostearate.

The appropriate fatty acid monoester content in the external preparation of the present invention is 1 to 98 weight%, preferably 3 to 70 weight%, more preferably 5 to 60 weight%, further preferably 10 to 50 weight% with respect to the total preparation amount. With a small fatty acid monoester content of less than 1 weight%, cyclosporine tends to precipitate upon being applied to the lesion. From a viewpoint of reducing local irritation, the fatty acid monoester content is preferably 50 weight% or less.

The external preparation of the present invention may contain solubilizers other than the above, provided that such solubilizers do not interfere with the effects of the present invention.

Examples of such solubilizers include water, fatty acid diesters, glycerin fatty acid esters, polyethylene glycols, triacetin, oleyl alcohol, 2-ethyl-1,3-hexanediol, propylene glycol, dipropylene glycol, propylene carbonate, crotamiton, 1,3-butylene glycol, glycerin, isopropanol, light liquid paraffin, squalane, dimethylpolysiloxane, and ethylene glycol salicylate.

Preferably, the external preparation of the present invention does not contain ketones as a solubilizer.

"Ketones" according to the external preparation of the present invention means compounds represented by R(R')C=O (where R and R' each represent alkyl). Examples of such compounds include methyl ethyl ketone, acetone, and methyl isobutyl ketone.

In the external preparation of the present invention, the appropriate content of solubilizers other than the ethanol and the fatty acid monoester is 40 weight% or less, preferably 20 weight% or less, more preferably 10 weight% or less of the total solubilizer amount. In this specification, the total solubilizer weight excludes the weight of components that do not act as solubilizers.

In the external preparation of the present invention, the appropriate content of water is 40 weight% or less, or 35 weight% or less of the total solubilizer amount, and, preferably, the external preparation of the present invention is substantially free of water used as a solubilizer, because water lowers the solubility of cyclosporine in the solubilizer, or the transdermal absorption of cyclosporine.

As used herein, "substantially free of water used as a solubilizer" means that the water content is typically 10 weight% or less, preferably 5 weight% or less, more preferably 3 weight% or less, further preferably zero with respect to the total amount of the solubilizer dissolving cyclosporine. The solubilizer dissolving cyclosporine may be emulsified with water using a surfactant to prepare a cream or an emulsion lotion, provided that there is no mixing of the solubilizer and water.

The form of the external preparation of the present invention may be, for example, a liquid formulation, a cream, a lotion, or a gel. The preferred form is a liquid formulation.

In addition to the foregoing components, the external preparation of the present invention may contain additives commonly used in the field of external preparations, for example, such as surfactants, thickeners, stabilizers, preservatives, pH adjusters, and fragrance ingredients.

The external preparation of the present invention may be produced by using methods known in the field of external preparations. For example, in the case of a liquid formulation, the external preparation of the present invention may be produced by dissolving cyclosporine in a mixed solution containing ethanol, a fatty acid monoester, and optional additives.

The external preparation of the present invention can be safely used for humans and other mammals (for example, rodents such as mice, hamsters, guinea pigs, rats, and rabbits, and other animals including dogs, cats, goats, sheep, cows, pigs, and monkeys).

The external preparation of the present invention is useful for the treatment of, for example, alopecia, such as alopecia areata (including alopecia universalis, alopecia areata monolocularis, alopecia areata multilocularis), and other diseases such as psoriasis, atopic dermatitis, contact dermatitis, seborrheic dermatitis, and prurigo. The external preparation of the present invention is particularly useful as a hair growth promoting agent (particularly, a hair growth promoting agent for alopecia areata).

Alopecia areata is triggered by autoimmunity. It is therefore believed that the cyclosporine external preparation having both immunosuppressing effect and hair growth effect would provide a therapeutic agent that is effective for alopecia areata.

The dose of the external preparation of the present invention varies depending on the type of the disease to be treated, the seriousness of the disease, and the like. As an example, when used as a hair growth promoting agent, the external preparation of the present invention may be applied to the lesion twice a day, each in a dose of 0.1 µg/cm² to 200 µg/cm² in terms of cyclosporine.

### EXAMPLES

The present invention is described below in greater detail using Example, Comparative Examples, and Test Example. The present invention, however, is not limited in any way by the following Example, Comparative Examples, and Test Example.

The cyclosporine A, the anhydrous ethanol, and the isopropyl myristate used in the Examples below are the products from Huandong Zhongmei, Amakasu Chemical Industries (Japanese Pharmacopoeia anhydrous ethanol), and Nikko Chemicals Co., Ltd., respectively.

### [Test Example 1] In Vitro Hairless Mouse Skin Permeation Test

Liquid formulations obtained by mixing the components shown in Table 1 were tested in an in vitro hairless mouse skin permeation test conducted with a Franz vertical diffusion cell (vertical Franz cell, receptor volume: 7 mL, effective diffusion area: 1.77 cm²).

A hairless mouse (Laboskin, Hos-HR1, male, 7 weeks of age, Hoshino Laboratory Animals Inc.) was used as a permeable membrane. As a receptor solution, a 1% bovine serum albumin-containing PBS [Dulbecco's PBS (-), Nissui Pharmaceutical Co., Ltd.; bovine serum albumin, nacalai tesque] was used. The liquid formulations (10 µL each) were applied in the form of a permeable membrane, and the receptor solution was stirred at the maintained temperature of 32°C. After 24 hours from the application of the liquid formulation, the skin was cleaned, and the dermis was collected by heat separation (60°C, 1 min, dry incubation). The cyclosporine A concentration in the dermis was quantified by liquid chromatography-tandem mass spectrometry (LC/MS/MS).

As a result, as shown in Table 2, the liquid formulation of Example 1 had higher transdermal absorption than the liquid formulations of Comparative Examples 1 and 2.

**[Table 1]**

| Component | Formulation (weight%) | | |
|---|---|---|---|
| | Example 1 | Comparative Example 1 | Comparative Example 2 |
| Cyclosporine A | 2.5 | 2.5 | 2.5 |
| Anhydrous ethanol | 48.75 | 39.0 | 48.75 |
| Isopropyl myristate | 48.75 | - | - |
| Purified water | - | 58.5 | - |
| Diethyl sebacate | - | - | 48.75 |

**[Table 2]**

| Test preparation | Dermis cyclosporine A concentration (µg/gram tissue) |
|---|---|
| | Mean value |
| Example 1 | 73.22 |
| Comparative Example 1 | 23.58 |
| Comparative Example 2 | 13.41 |
| (n = 3) | |

## Claims

1. An external preparation comprising cyclosporine, ethanol, and a fatty acid monoester.

2. The external preparation according to claim 1, wherein the fatty acid monoester is an ester of a monohydric alcohol of 1 to 22 carbon atoms and a monocarboxylic acid of 6 to 22 carbon atoms.

3. The external preparation according to claim 1, wherein the fatty acid monoester is isopropyl myristate, isopropyl palmitate, or 2-hexadecyl isostearate.

4. The external preparation according to any one of claims 1 to 3, which is substantially free of water used as a solubilizer.

5. The external preparation according to any one of claims 1 to 4, which is a liquid formulation.
